# EUROPÄISCHE PATENTSCHRIFT

(11) **EP 2 314 234 B1**
(45) Veröffentlichungstag und Bekanntmachung des Hinweises auf die Patenterteilung: **27.03.2013**
(21) Anmeldenummer: 10013848.6
(22) Anmeldetag: 21.10.2010
(51) Int. Cl.: A61B 17/29, A61B 17/16

(54) **Chirurgisches Instrument**
Surgical instrument
Instrument chirurgical

(30) Priorität: 22.10.2009 DE 202009014310 U
(43) Veröffentlichungstag der Anmeldung: 27.04.2011
(73) Patentinhaber: Thomas Tontarra Grundstücksverwaltungs GmbH & Co. KG, 78573 Wurmlingen (DE)
(72) Erfinder: Petrella, Alberto, 78573 Wurmlingen (DE); Tontarra, Thomas, 78573 Wurmlingen (DE)
(74) Vertreter: Maser, Jochen

(56) Entgegenhaltungen:
- DE-U1-202009 001 811

## Beschreibung

Die Erfindung betrifft ein chirurgisches Instrument, insbesondere ein Schiebeschaftinstrument, mit einem Hauptteil und einem relativ dazu bewegbaren Teil, mit einem an dem Hauptteil angeordneten Handgriff, der einen feststehenden Griff und einen das bewegbare Teil betätigbaren Griff aufweist, mit zumindest einer Führung, durch welches das bewegbare Teil relativ zum Hauptteil zwischen einer Ausgangsposition und einer Arbeitsposition bewegbar ist und mit einer am bewegbaren Teil angeordneten und zum Hauptteil weisenden Gleitfläche, die entlang einer am Hauptteil angeordneten und zur Gleitfläche weisenden Führungsfläche verschiebbar geführt ist.

Aus der DE 20 2009 001 811 U1 ist ein Schiebeschaftinstrument mit einem Hauptteil und einem relativ dazu bewegbaren Teil bekannt. An dem

Hauptteil ist ein Handgriff angeordnet, der einen feststehenden Griff und einen das bewegbare Teil betätigbaren Griff umfasst. Das bewegbare Teil ist durch eine proximale Führung zum Hauptteil bewegbar geführt, so dass das bewegbare Teil durch den Handgriff zwischen einer Arbeitsposition und einer Ausgangsposition verschiebbar ist. Das bewegbare Teil ist über eine Führung nahe der Schneide mit dem Hauptteil verbunden. Gegenüberliegend ist eine Gelenkverbindung vorgesehen, an welcher der bewegbare Handgriff an dem bewegbaren Teil angreift. Dazwischen liegend ist ein Reinigungsspalt ausgebildet, der eine flache Vertiefung an der Unterseite des bewegbaren Teils umfasst. Diese Vertiefung erstreckt sich über die gesamte Breite des Schaftteils quer zur Längsachse des Schiebeschaftinstrumentes. Dadurch ist eine Unterseite des bewegbaren Teils mit einem Spaltabstand zur gegenüberliegenden Seite des Hauptteils beabstandet. Diese Anordnung ermöglicht eine Reinigung zwischen dem bewegbaren Teil und dem Hauptteil im Bereich zwischen der Führung der Gelenkverbindung.

Der Erfindung liegt die Aufgabe zugrunde, ein chirurgisches Instrument, insbesondere Schiebeschaftinstrument, zu schaffen, welches eine Reinigung und Sterilisation verbessert, ohne dass das chirurgische Instrument auch nur teilweise für den Reinigungsprozess zerlegt werden muss.

Diese Aufgabe wird durch ein chirurgisches Instrument gemäß den Merkmalen des Anspruchs 1 gelöst. Weitere vorteilhafte Ausführungsformen und Weiterbildungen sind in weiteren abhängigen Ansprüchen angegeben.

Bei der erfindungsgemäßen Ausgestaltung des chirurgischen Instrumentes, bei welchem zumindest die Gleitfläche des bewegbaren Teils oder die Führungsfläche des Hauptteils zur Bildung eines Spaltes zumindest abschnittsweise aufeinander aufliegen und zumindest die Gleitfläche oder die Führungsfläche quer zur Längserstreckung des Hauptteils und des bewegbaren Teils auf eine Breite reduziert ist oder bei welchen zumindest die Gleitfläche oder Führungsfläche durch zumindest eine quer zur Längserstreckung des Hauptteils und bewegbaren Teils sich erstreckende Aussparung durchbrochen ist, so dass ein spülbarer Spalt zwischen der Gleitfläche und der Führungsfläche zur Reinigung des chirurgischen Instrumentes gebildet wirdweist den Vorteil auf, dass nach dem Gebrauch des chirurgischen Instruments keine weiteren Handgriffe erforderlich sind, um das chirurgische Instrument zu zerlegen, damit dieses chirurgische Instrument gereinigt und sterilisiert werden kann. Eine anschließende Montage zu dem nächsten Einsatz entfällt auch. Es ist somit eine zerlegungsfreie Reinigung des chirurgischen Instruments möglich.

Des Weiteren weist die erfindungsgemäße Ausgestaltung den Vorteil auf, dass durch die Verringerung der aneinander grenzenden Gleit- und Führungsflächen auch eine Verringerung der Gleitreibungskraft zwischen dem Hauptteil und dem bewegbaren Teil ermöglicht wird, wodurch eine Verbesserung der Laufeigenschaften beziehungsweise eine leichtgängigere Bewegung des bewegbaren Teils zum Hauptteil gegeben ist. Analoges gilt für die Zuordnung zueinander unter Bildung eines spülbaren Spaltes. Gleiches gilt für eine beliebige Kombination dieser Alternativen. Die erfindungsgemäße Ausführungsform ermöglicht des Weiteren, dass die kritischen Bereiche eines chirurgischen Instrumentes, in welchen sich die Bakterien ansammeln, zugänglich sind oder in einer Waschmaschine mit Waschwasser gereinigt und vorzugsweise anschließend sterilisiert werden können. Dadurch ist die Zerlegung des chirurgischen Instrumentes entbehrlich.

Eine weitere bevorzugte Ausgestaltung der Erfindung sieht vor, dass zumindest die Gleitfläche oder die Führungsfläche in einer Raumrichtung durchbrochen ist, die in Längsrichtung des Hauptteils und bewegbaren Teils liegt, wobei die Raumrichtung bevorzugt in einer Trennebene zwischen dem Hauptteil und bewegbaren Teil liegt. Dies stellt eine einfache Ausführungsform dar und ermöglicht die Beibehaltung der bisherigen Funktionsweise des chirurgischen Instrumentes. Bei dieser Ausführungsform erfolgt dies insbesondere durch die Durchbrechung zumindest der Führungsfläche oder Gleitfläche zusätzlich zur Aussparung.

Eine weitere bevorzugte Ausgestaltung der Erfindung sieht vor, dass zumindest die Gleitfläche oder die Führungsfläche zur Bildung eines spülbaren Spaltes einander zugeordnet sind. Dadurch kann unter anderem bei einem Spülvorgang eine Düsenwirkung erzielt wird, um die Reinigung zu verbessern. Bei dieser Ausführungsform sind zumindest die aneinandergrenzenden Flächenabschnitte zwischen der Gleitfläche und der Führungsfläche so gering bemessen, dass beim Reinigen des chirurgischen Instruments in einer Waschmaschine die im Reinigungsprozess verwendeten Sprühstrahlen genügen, um ggf. in diesem Spalt sich befindliche Bakterien oder Verunreinigungen auszuwaschen.

Nach einer bevorzugten Ausgestaltung der Erfindung ist zumindest an einer Außenseite des Hauptteils oder des bewegbaren Teils zumindest eine an die Führungsfläche oder Gleitfläche angrenzende Schrägfläche vorgesehen, welche zumindest abschnittsweise zur Längsmittelachse geneigt ist. Dadurch wird die Breite der Gleitfläche und/oder der Führungsfläche reduziert. Beispielsweise bei einer an dem bewegbaren Teil angeordneten Schrägfläche wird die zwischen der Gleitfläche und der Schrägfläche gebildete Kante gegenüber der Kante zwischen der Führungsfläche und einer Außenseite des Hauptteils nach innen bzw. zur Längsmittelachse hin versetzt, so dass im Querschnitt gesehen ein Rücksprung, insbesondere ein dreiecksförmiger Rücksprung, entsteht. Dadurch wird beim Auftreffen eines Sprühstrahles auf das chirurgische Instrument der Sprühstrahl zum Spalt zwischen der Gleitfläche und der Führungsfläche hin geführt, so dass ein verbessertes Spülen des Spaltes oder der Durchbrechung beziehungsweise Aussparung ermöglicht wird. Diese Wirkung wird insbesondere dann verbessert, wenn symmetrisch zur Spaltebene, die durch die Gleit- und Führungsflächen gebildet wird, eine solche Schrägfläche angeordnet ist. Dadurch kann der Sprühstrahl gezielt zum Spalt hingeführt werden. Gleichzeitig entstehen bei der Benutzung eines solchen chirurgischen Instruments keine Beschädigungen an dem am Schaft angrenzenden Gewebe.

Nach einer weiteren bevorzugten Ausgestaltung der Erfindung ist an jeder Außenseite der Gleit- oder Führungsfläche angrenzend eine Schrägfläche vorgesehen und diese Schrägfläche ist vorzugsweise spiegelsymmetrisch zur Längsmittelachse und insbesondere zum Spalt ausgebildet. Dadurch kann beispielsweise im Querschnitt gesehen eine kegelstumpfförmige Geometrie ausgebildet sein, wodurch die Spaltbreite zwischen der Gleitfläche und der Führungsfläche gegenüber chirurgischen Instrumenten gemäß dem Stand der Technik erheblich reduziert werden kann. Dabei können die reduzierte Breite der Schrägfläche und diese reduzierte Breite der Führungsfläche gleich oder auch voneinander abweichend vorgesehen sein.

Des Weiteren ist bevorzugt an nur einer Außenseite eine Schrägfläche vorgesehen, welche sich im Wesentlichen über die gesamte Breite des Hauptteils oder bewegbaren Teils erstreckt, so dass die Gleitfläche und Führungsfläche direkt an eine Außenseite angrenzt und in der Breite auf einen spülbaren Spalt reduziert ist. Diese Ausgestaltung weist den Vorteil auf, dass eine maximale Größe eines Mündungsbereiches oder Zuführbereiches für die Sprühflüssigkeit im noch verbleibenden Spalt zwischen dem Hauptteil und dem bewegbaren Teil gegeben ist. Darüber hinaus kann die gegenüberliegende Außenseite nahezu geschlossen ausgebildet werden.

Eine weitere bevorzugte Ausgestaltung der Erfindung sieht vor, dass zumindest die Gleitfläche oder Führungsfläche durch eine quer zur Längserstreckung des Hauptteils und bewegbaren Teils sich erstreckende Aussparung durchbrochen ist. Durch solche Aussparungen wird der zwischen dem bewegbaren Teil und Hauptteil gebildete Spalt vollständig unterbrochen. Somit ist dieser Bereich besonders einfach zu reinigen. Bevorzugt sind zwischen den Führungen und/oder Gelenkverbindungen und insbesondere in Längserstreckung des Hauptteils und bewegbaren Teils sich erstreckende Aussparungen zumindest abschnittsweise in der Gleitfläche und/oder der Führungsfläche vorgesehen.

Eine alternative Ausgestaltung der Erfindung sieht vor, dass zumindest die Gleitfläche oder die Führungsfläche durch eine in Längsrichtung des Hauptteils und bewegbaren Teils sich erstreckende Aussparung durchbrochen ist. Diese alternative Ausgestaltung weist den Vorteil auf, dass das chirurgische Instrument, insbesondere das Schiebeschaftinstrument, das bisherige Erscheinungsbild von herkömmlichen chirurgischen Instrumenten beibehalten kann, d. h., dass die Außenseiten des beweglichen Teils und des Hauptteils bündig ineinander übergehen, jedoch die Vorteile der Erfindung gegeben sind. Dabei kann beispielsweise eine Aussparung in Form eines Längskanals oder Durchgangskanals gebildet werden, wodurch die Reinigungs- und Spülflüssigkeit am proximalen oder distalen Ende eingeführt und auf der jeweils gegenüberliegenden Seite herausgeführt wird. Gleichzeitig kann ein seitliches Ausdringen über den verbleibenden und spülbaren Spalt zwischen der Gleitfläche und den Führungsflächen erfolgen.

Eine vorteilhafte Weiterbildung der Erfindung sieht vor, dass die Aussparung wellenförmig ausgebildet ist. Dadurch können große Bereiche an der Gleit- oder Führungsfläche zur gegenüberliegenden Fläche oder an beiden Flächen ausgespart werden und somit für die Reinigung gut zugänglich sein. Alternativ können die Aussparungen dreiecksförmig, rechteckförmig, trapezförmig, bogensegmentförmig oder sägezahnförmig ausgebildet sein. Solche Aussparungen werden insbesondere dadurch gebildet, indem diese sich von der Gleit- oder Führungsfläche aus in das bewegbare Teil oder das Hauptteil vertiefen. Diese Ausgestaltung ermöglicht, dass die gegenüberliegende Außenseite des bewegbaren Teils oder Hauptteils eine geschlossene Oberfläche aufweist und ein hinreichender Vollquerschnitt verbleibt, um die Kräfte zu schneiden oder das Stanzen von Gewebe oder dergleichen zu ermöglichen. Die Aussparungen können einheitlich sein oder auch miteinander kombiniert werden.

Eine alternative Ausgestaltung für die Aussparungen sieht vor, dass diese zwischen zwei benachbart zu einander angeordneten kegelstumpfförmigen, pyramidenstumpfförmigen, halbkugelförmigen oder bogensegmentförmigen Erhebungen ausgebildet sind. Dadurch sind weitere geometrische Alternativen zur Herstellung der Aussparung gegeben, die sowohl ein leichtes Reinigen als auch eine geringere Kraft zur Betätigung des chirurgischen Instrumentes ermöglichen.

Nach einer weiteren bevorzugten Ausgestaltung der Erfindung ist vorgesehen, dass zumindest eine Aussparung oder eine Schrägfläche an eine Führung angrenzt. Die Führung zwischen dem bewegbaren Teil und dem Hauptteil kann dabei darin bestehen, dass beispielsweise eine T-förmige Führungsnut oder eine ähnliche geometrische Führungsnut einerseits und ein komplementär ausgebildeter Führungszapfen andererseits vorgesehen sind. Des Weiteren kann die Führung durch eine Gelenkverbindung oder ein Doppelgelenk zwischen einem betätigbaren Griff und dem bewegbaren Teil gebildet sein. Durch die Angrenzung der Aussparung oder der Schrägfläche an die Führung wird sichergestellt, dass diese Bereiche ebenfalls für die Reinigungsflüssigkeit und die anschließende Desinfektion zugänglich sind.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
- Figur 1: eine schematische Seitenansicht eines erfin- dungsgemäßen chirurgischen Instrumentes,
- Figuren 2a bis c: schematisch vergrößerte Seiten- und Schnitt- ansichten des chirurgischen Instrumentes gemäß Figur 1,
- Figuren 3a und b: eine schematische vergrößerte Seiten- und Schnittansicht einer alternativen Ausführungs- form zu den Figuren 2a und b,
- Figuren 4a und b: eine schematische vergrößerte Seiten- und Schnittansicht einer alternativen Ausführungs- form zu den Figuren 2a und b,
- Figuren 5a und b: eine schematische vergrößerte Seiten- und Schnittansicht einer alternativen Ausführungs- form zu den Figuren 2a und b,
- Figuren 6a und b: eine schematische vergrößerte Seiten- und Schnittansicht einer alternativen Ausführungs- form zu den Figuren 2a und b,
- Figuren 7a und b: eine schematische vergrößerte Seiten- und Schnittansicht einer alternativen Ausführungs- form zu den Figuren 2a und b,
- Figuren 8a und b: eine schematische vergrößerte Seiten- und Schnittansicht einer alternativen Ausführungs- form zu den Figuren 2a und b,
- Figuren 9a und b: eine schematische vergrößerte Seiten- und Schnittansicht einer alternativen Ausführungs- form zu den Figuren 2a und b und
- Figuren 10a bis d: schematische Ansichten einer alternativen Ausführungsform zum chirurgischen Instru- ment gemäß den Figuren 1, 2a bis c.

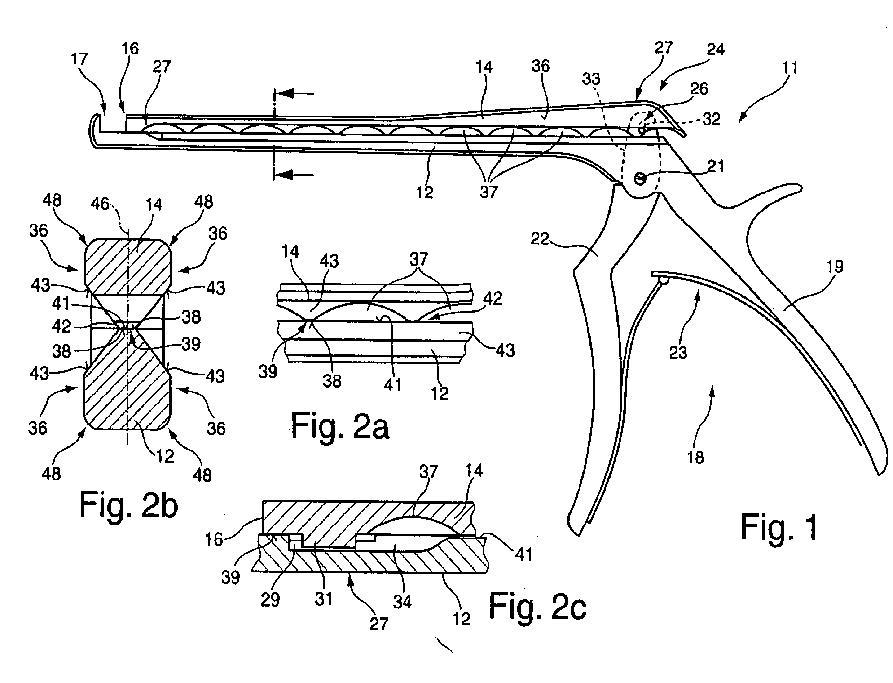

Eine erste erfindungsgemäße Ausführungsform eines chirurgischen Instrumentes 11 ist in den Figuren 1, 2a bis c dargestellt. Bei diesem chirurgischen Instrument 11 handelt es sich beispielsweise um ein Schiebeschaftinstrument, eine sogenannte Stanze, insbesondere Kerrison-Stanze, welche bei chirurgischen Eingriffen für die Entfernung von Gewebeknochen oder dergleichen eingesetzt wird. Die Erfindung ist nicht auf diese Stanzen beschränkt, sondern kann auf alle chirurgischen Instrumente übertragen werden, welche dieselbe Problematik hinsichtlich der Reinigung und Sterilisation aufweisen.

Das chirurgische Instrument 11 weist ein Hauptteil 12 auf, welches auch Schaft bezeichnet wird. Dieses Hauptteil 12 nimmt ein bewegbares Teil 14 auf, das relativ zum Hauptteil 12 verschiebbar ist. Das bewegbare Teil 14 wird auch Schieber genannt. Am proximalen Ende des Hauptteils 12 und des beweglichen Teils 14 sind Funktionselemente 16, 17 vorgesehen. Beispielsweise kann dies eine Schneide und eine Gegenplatte oder Gegenschneide sein. Ebenso kann dies bei anderen Ausführungsformen ein scherenförmiges Schneidwerkzeug, ein löffelförmiges Schneidwerkzeug, eine Fasszange oder dergleichen sein. Die Ausgestaltung der Funktionselemente 16, 17 ist anwendungsspezifisch für den chirurgischen Eingriff vorgesehen.

An dem Hauptteil 12 ist ein Handgriff 18 angeordnet. Das Hauptteil 12 geht in einen feststehenden Griff 19 über und weist im Übergangsbereich vom Hauptteil 12 zum feststehenden Griff 19 eine Drehachse 21 auf, um welche ein betätigbarer Griff 22 schwenkbar angeordnet ist. Der betätigbare Griff 22 und feststehende Griff 19 werden durch ein Federelement 23 in einer Ausgangsposition 24 zueinander angeordnet, wie dies in Figur 1 dargestellt ist. In dieser Ausgangsposition 24 sind beispielsweise die Funktionselemente 16, 17 zueinander beabstandet. Unter Handgriff 18 kann sowohl die dargestellte Griffart als auch ein Ringgriff oder weitere Griffvarianten verstanden werden.

Bei Betätigung des Griffs 22 wird dieser um die Drehachse 21 geschwenkt, wobei das bewegbare Teil 14 nach links bewegt wird, um das Funktionselement 16 auf das Funktionselement 17 zuzubewegen. Sobald diese beispielsweise aneinander anliegen, ist eine Arbeitsposition erreicht. Sobald der betätigbare Handgriff 18 gelöst wird, wird das bewegbare Teil 14 aufgrund der Federkraft des Federelementes 23 selbständig in die Ausgangsposition 24 zurückgeführt.

Das bewegbare Teil 14 ist durch eine Gelenkverbindung 26 am distalen Ende des chirurgischen Instrumentes 11 geführt und weist beispielsweise am proximalen Ende eine Führung 27 auf, welche in Figur 2c näher dargestellt ist. Alternativ kann nur im mittleren Bereich des Hauptteils 12 und bewegbaren Teils 14 oder auch zusätzlich eine Führung vorgesehen sein. Beispielsweise ist als Führung 27 am Hauptteil 12 eine T-nutenförmige Vertiefung 29 vorgesehen, in welche ein Führungszapfen 31 eingreift und darin längsverschieblich geführt ist. An die Vertiefung 29 angrenzend ist ein Einführungsbereich 34 vorgesehen, der ein Einsetzen des Führungszapfens 31 in die Vertiefung 29 bei der Komplettierung des bewegbaren Teils 14 zum Hauptteil 12 ermöglicht. Nachdem das bewegbare Teil 14 zum Hauptteil 12 positioniert ist, wird die weitere Führung 27 beispielsweise als Gelenkverbindung 26 hergestellt, indem beispielsweise ein Zapfen oder eine Schraube durch das bewegbare Teil 14 unter Hindurchführung durch eine Ausnehmung 32, insbesondere eines Langloches, in einer Lasche 33 des betätigbaren Griffes 22 erfolgt. Im Anschluss daran kann der Zapfen oder die Schraube mit einer Außenseite 36 verschliffen werden. Eine lösbare Anordnung der Gelenkverbindung 26 zum Abnehmen des bewegbaren Teils 14 vom Hauptteil 12 zur Reinigung und Sterilisation des chirurgischen Instrumentes 11 ist nicht möglich und aufgrund der erfindungsgemäßen Ausgestaltung des chirurgischen Instrumentes auch nicht erforderlich.

Eine erste erfindungsgemäße Ausführungsform des chirurgischen Instruments 11 ist in den Figuren 2a, 2b und 2c vergrößert dargestellt. Das Hauptteil 12 weist eine zum bewegbaren Teil 14 weisende Führungsfläche 41 auf. Dieser gegenüberliegend ist eine Gleitfläche 39 am bewegbaren Teil 14 zugeordnet. Während einer Verschiebebewegung des bewegbaren Teils 14 relativ zum Hauptteil 12 kann die Gleitfläche 39 entlang der Führungsfläche 41 geführt werden, wobei die Gleitfläche 39 und Führungsfläche 41 unter Bildung eines geringen Spaltes 42 zumindest abschnittsweise aufeinander aufliegen oder auch vollständig aneinander anliegen können. Das bewegbare Teil 14 weist bei dieser ersten Ausführungsform beispielsweise eine Gleitfläche 39 auf, die zumindest in einer Raumrichtung durchbrochen ist. In diesem Fall ist das bewegbare Teil 14 quer zur Längserstreckung des bewegbaren Teils 14 durch eine Aussparung 37 durchbrochen beziehungsweise unterbrochen. Diese Aussparung 37 ist gemäß Figur 2a bogenförmig ausgebildet. Bevorzugt sind mehrere Aussparungen 37 aneinander gereiht, so dass diese Aussparungen 37 sich von einem proximalen Ende des bewegbaren Teiles 14 bis zum distalen Endes bewegbaren Teiles 14 erstrecken. Dabei ist bevorzugt vorgesehen, dass die proximale Aussparung zumindest bis an die Führung 27 heranreicht oder diese zumindest teilweise von außen zugänglich macht. Analoges gilt im distalen Bereich für die Gelenkverbindung 26, so dass auch diese zum Reinigen gut zugänglich ist.

Zwischen den Aussparungen 37 sind Flächenabschnitte 38 der Gleitfläche 39 des bewegbaren Teils 14 ausgebildet. Die Gleitfläche 39 ist durch diese Flächenabschnitte 38 gegenüber herkömmlichen chirurgischen Instrumenten erheblich reduziert, bei denen eine durchgehende Gleitfläche 39 zwischen dem proximalen und distalen Ende des bewegbaren Teils 14 vorgesehen ist. Der Gleitfläche 39 gegenüberliegend ist am Hauptteil 12 eine Führungsfläche 41 vorgesehen. Im Ausführungsbeispiel ist diese Führungsfläche 41 durchgehend ausgebildet mit Ausnahme der Vertiefung 29 und der an die Vertiefung 29 angrenzenden Einführbereich 34 und einem distalen Endbereich, in welchem die Lasche 33 des betätigbaren Griffes 22 zur Betätigung des chirurgischen Instrumentes 11 angeordnet ist. Alternativ kann vorgesehen sein, dass die Führungsfläche 41 analoge Aussparungen 37 aufweist oder die Gleitfläche 39 und die Führungsfläche 41 Aussparungen 37 aufweist. Alternativ kann vorgesehen sein, dass das bewegbare Teil 14 eine durchgehende Gleitfläche 39 und die Aussparungen 37 nur an der Führungsfläche 41 vorgesehen sind. Ebenso kann dies auch alternierend vorgesehen sein, so dass in einem Bereich Aussparungen 37 an der Führungsfläche 41, anschließend ein Bereich mit Aussparungen 37 an der Gleitfläche 39 und wieder anschließend ein Bereich mit Aussparungen an der Führungsfläche 41 vorgesehen ist. Solche Kombinationen und Anordnungen sind beliebig auswählbar.

Durch die Ausgestaltung solcher Aussparungen 37 wird erzielt, dass die dazwischen liegenden Flächenabschnitte 38 zumindest in der Länge in Längsrichtung des Hauptteils 12 gesehen sehr gering sind, wodurch nur sehr schmale Spalte 42 zwischen den Flächenabschnitten 38 der Gleitfläche 39 und der Führungsfläche 41 gebildet sind. Dadurch wird eine leichte und gute Reinigung ermöglicht. Gleichzeitig wird ein Ausspülen des Spaltes 42 erzielt. Darüber hinaus ist eine optische Prüfung auf Sauberkeit gegeben. Zusätzlich weist ein solches chirurgisches Instrument 11 gute Laufeigenschaften auf. Bevorzugt sind die Gleit- und/oder Führungsflächen 39, 41 auf eine Breite von weniger als 70 %, insbesondere weniger als 90 % bezogen auf die Breite des Hauptteils 12 oder des bewegbaren Teils 14 oder von beiden reduziert. Es kann auch eine punkt- oder linienförmige Zuordnung der Gleit- und Führungsflächen 39, 41 zueinander vorgesehen sein.

Ergänzend zu diesen Aussparungen 37 kann ein- oder beidseitig an der Außenseite 36 des bewegbaren Teils 14 und/oder des Hauptteils 12 eine Schrägfläche 43 vorgesehen sein, welche in Abhängigkeit der Neigung die verbleibende Breite der Flächenabschnitte 38 der Gleitfläche 39 bestimmt. Bevorzugt sind solche Schrägflächen 43 symmetrisch zur Mittelachse 46 des Hauptteils 12 und bewegbaren Teils 14 vorgesehen, so dass beide Außenseiten 36 in gleichem Maße ausgebildet sind. Sofern die Schrägflächen 43 beispielsweise nur an dem bewegbaren Teil 14 angeordnet sind, entsteht ein dreiecksförmiger Freischnitt, wodurch beim Absprühen des chirurgischen Instrumentes 11 eine Art Zuführkanal zur Durchspülung des Spaltes 42 gegeben ist. Die vorbeschriebene Anordnung kann analog auch nur an dem Hauptteil 12 vorgesehen sein. Die bevorzugte Ausführungsform gemäß Figur 2b zeigt, dass die Schrägflächen nicht nur symmetrisch zur Mittelachse 46, sondern auch am Hauptteil 12 und bewegbaren Teil 14 angeordnet sind. Dadurch kann eine besonders bevorzugte trichterförmige Anordnung zur Zuführung des Reinigungsmediums zur Spülung des Spaltes 42 gegeben. Der Verlauf der Schrägfläche 43 als Gerade ist nur beispielhaft. Weitere Geometrien sind ebenso möglich.

In Figur 2b ist beispielsweise die Breite der Führungsfläche 41, die vorzugsweise durchgängig vom distalen bis proximalen Ende des Hauptteils 12 ausgebildet ist, gleich breit wie die Breite der Flächenabschnitte 38 der Gleitfläche 39 ausgebildet. Alternativ kann eine der beiden Flächen 39, 41 in der Breite größer oder kleiner ausgebildet sein. Darüber hinaus kann vorgesehen sein, dass beispielsweise eine der beiden Flächen 39, 41 nicht eben ausgebildet ist, so dass beispielsweise ebene Flächenabschnitte 38 der Gleitfläche 39 an einer gerundeten Führungsfläche 41 anliegen.

Die dargestellten Querschnitte des bewegbaren Teils 14 und des Hauptteils 12 sind nur beispielhaft als quadratischer oder rechteckförmiger Flächenabschnitt mit gerundeten Außenkanten 48 dargestellt. Diese Geometrien können beliebig ausgestaltet werden.

Eine alternative nicht näher dargestellte Ausführungsform sieht vor, dass das Hauptteil 12 und bewegbare Teil 14 jeweils einen Querschnitt gemäß Figur 2b aufweisen, wobei die Aussparungen 37 nicht eingebracht sind. Auch eine solche alternative Ausführungsform ermöglicht, dass das chirurgische Instrument 11 ohne Zerlegung vollständig zu reinigen und zu sterilisieren ist. Der zwischen der in Längsrichtung durchgehenden Gleitfläche 39 und ebenso durchgehenden Führungsfläche 41 sich bildende Spalt 42 ist derart gering und spülbar, dass gegebenenfalls sich darin ansammelnde Bakterien herausgespült werden können. Des Weiteren ist bevorzugt vorgesehen, dass die Breite der Gleitfläche 39 geringer als die Breite des Einführungsbereiches 34 ausgebildet ist, so dass auch dieser Bereich und die Führung 27 selbst gespült werden kann. Zusätzlich können die Führungen 27 zur verschiebbaren Anordnung des bewegbaren Teils 14 zum Hauptteil 12 derart angeordnet sein, dass eine geringe Spalthöhe ausgebildet ist.

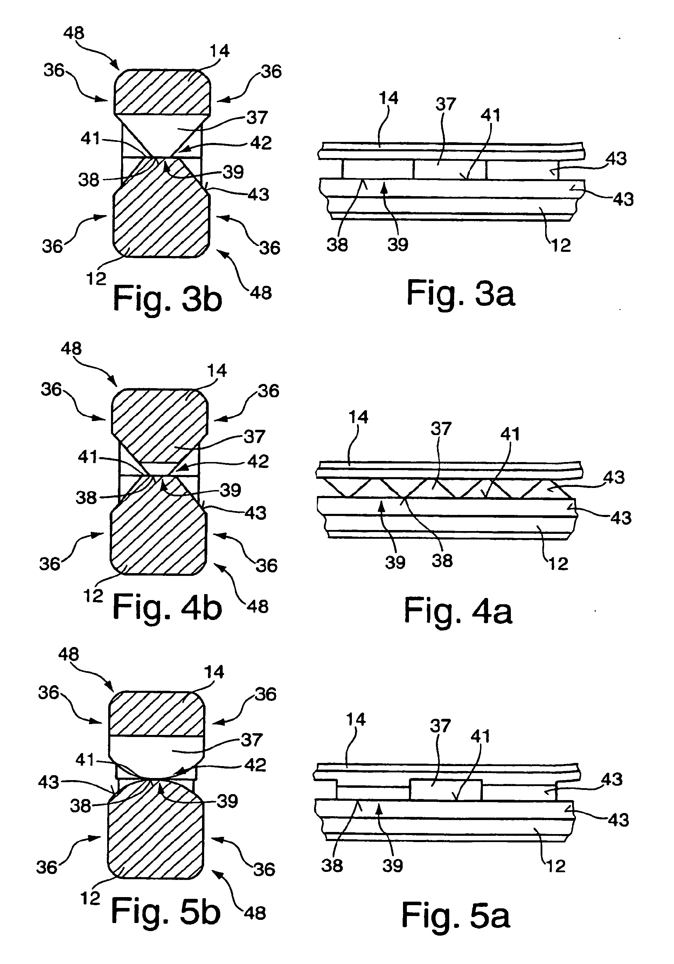

In den Figuren 3a und b ist eine alternative Ausführungsform zu den Figuren 2a und b dargestellt. Bei dieser Ausführungsform ist vorgesehen, dass die Aussparung 37 beziehungsweise die Aussparungen 37 rechteckförmig ausgebildet sind. Die zwischen den Aussparungen 37 verbleibenden Flächenabschnitte sind aufgrund der daran angeordneten Schrägflächen 43 wiederum gering ausgebildet. Es werden dann beispielsweise schmale, in Längserstreckung des chirurgischen Instrumentes 11 ausgerichtete rechteckförmige Flächenabschnitte 38 der Gleitfläche 39 ausgebildet. Die Führungsfläche 41 ist beispielsweise breiter als die Flächenabschnitte 38 der Gleitfläche 39 ausgebildet.

In den Figuren 4a und b ist eine weitere alternative Ausführungsform zu den Figuren 2a und b dargestellt. Bei dieser Ausführungsform ist vorgesehen, dass die Aussparung 37 zick-zack-förmig beziehungsweise dreiecksförmig ausgebildet ist. Dadurch kann wiederum ein hinreichend großer Freiraum zwischen dem bewegbaren Teil 14 und Hauptteil 12 gebildet werden, um spülbare Spalte 42 zwischen den Flächenabschnitten 38 der Gleitfläche 39 und der Führungsfläche 41 auszubilden.

In den Figuren 5a und b ist eine weitere alternative Ausführungsform zu den Figuren 2a und b dargestellt. Dabei ist vorgesehen, dass anstelle der geradlinig verlaufenden Schrägflächen 43 eine bogensegmentförmige Rundung ausgebildet ist, so dass die Flächenabschnitte 38 der Gleitfläche 39 quasi linienförmig an der Führungsfläche 41 anliegen. Dazwischen liegend sind wiederum Aussparungen 37 ausgebildet, die in diesem Ausführungsbeispiel als rechteckförmige Aussparungen 37 dargestellt sind. Alternativ können ebenso bogenförmige, dreiecksförmige oder auch weitere geometrische Formen vorgesehen sein.

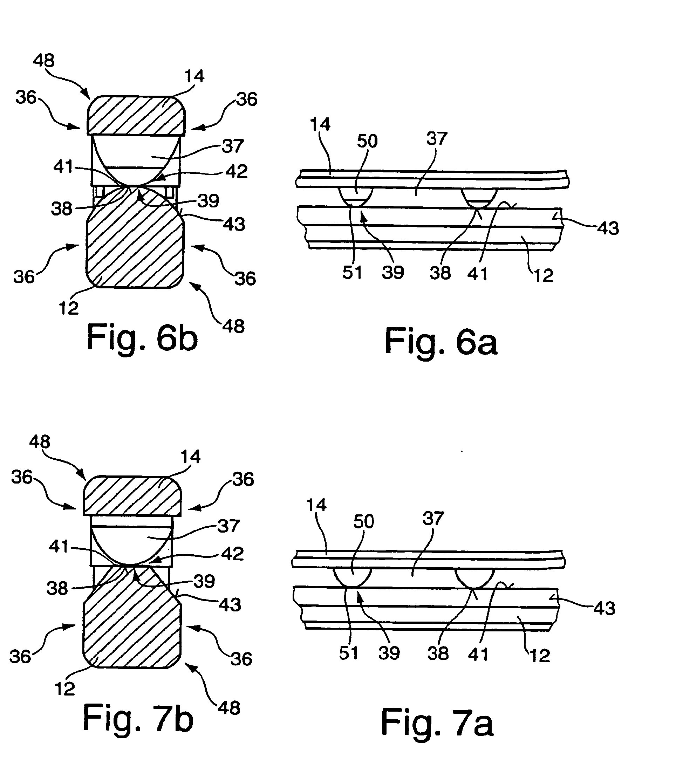

In den Figuren 6a und b ist eine weitere alternative Ausführungsform zu den Figuren 2a und b dargestellt. Bei dieser Ausführungsform ist vorgesehen, dass die Aussparung 37 durch zwei kegelstumpfförmige Erhebungen 50 gebildet ist, welche eine gerundete Spitze 51 aufweisen, an denen der Flächenabschnitt 38 der Gleitfläche 39 vorgesehen ist, welcher über der Führungsfläche 41 dem Hauptteil 12 gegenüberliegt. Die Führungsfläche 41 ist dabei von der Außenseite 36 beginnend mit zunächst einer Schrägfläche 43 ausgestattet, wobei die beiden von außen nach innen geneigten Schrägflächen 43 durch eine Rundung oder eine kuppelförmige Ausgestaltung der Führungsfläche 41 miteinander verbunden sind.

In den Figuren 7a und b ist eine alternative Ausführungsform zu den Figuren 6a und b dargestellt. Dabei ist vorgesehen, dass die Erhebungen 50 halbkreisförmig ausgebildet sind und die Führungsfläche 41 als ebene Fläche vorliegt. Die Breite der Führungsfläche 41 des Hauptteils 12 ist durch die Schrägflächen 43 wiederum begrenzt.

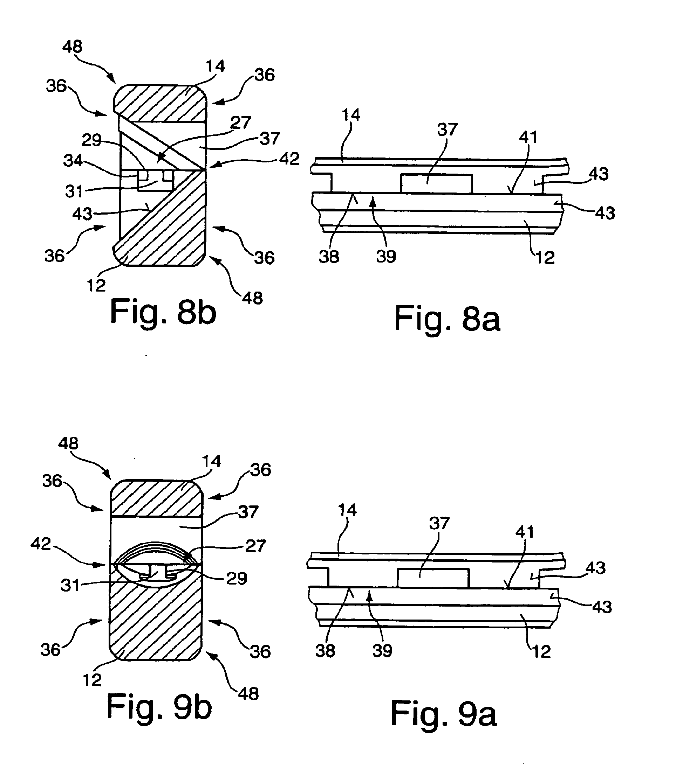

In den Figuren 8a und b ist eine weitere alternative Ausführungsform vorgesehen. Diese Ausführungsform ist geprägt durch eine Schrägfläche 43, welche sich an dem bewegbaren Teil 14 von einer Außenseite 36 bis quasi zur anderen Außenseite 36 erstreckt. Dazwischen können noch Aussparungen 37 mit verschiedener Geometrie vorgesehen sein. Sofern an dem bewegbaren Teil 14 und bevorzugt auch an dem Hauptteil 12 eine solche Schrägfläche 43 vorgesehen ist, verringert sich der verbleibende Spalt 42 auf ein Minimum, wobei der Spalt an einer Außenseite 36 des Hauptteils 12 und bewegbaren Teils 14 angrenzt. Dadurch wird gleichzeitig ermöglicht, dass die Vertiefung 29 der Führung 27 frei zugänglich ist. Diese Ausgestaltung kann ebenso um 180° gedreht angeordnet sein.

In den Figuren 9a und b ist eine weitere alternative Ausführungsform vorgesehen. Diese Ausführungsform weist eine konkave Gleitfläche 39 und eine konkave Führungsfläche 41 auf, wodurch zwei an die Außenseite 36 angrenzende Spalte 42 gebildet sind. Zur Spülung eines solchen mittleren Kanals sind bevorzugt wiederum quer verlaufende Aussparungen 37 vorgesehen, die beispielsweise als rechteckförmige Aussparungen 37 ausgebildet sind. Es versteht sich, dass auch nur eine der beiden Flächen 39, 41 eine solche konkave Ausgestaltung aufweisen kann. Ebenso können weitere geometrische Vertiefungen eingebracht werden, um einen solchen analogen Aufbau gemäß der Schnittdarstellung in Figur 9b zu erzielen.

Die vorstehenden Ausführungsformen zeigen, dass beliebige Kombinationen in der Geometrie der Führungsflächen 41 und der Gleitfläche 39 möglich sind, um einen spülbaren Spalt 42 auszubilden, der zwischen den Aussparungen 37 verbleibt.

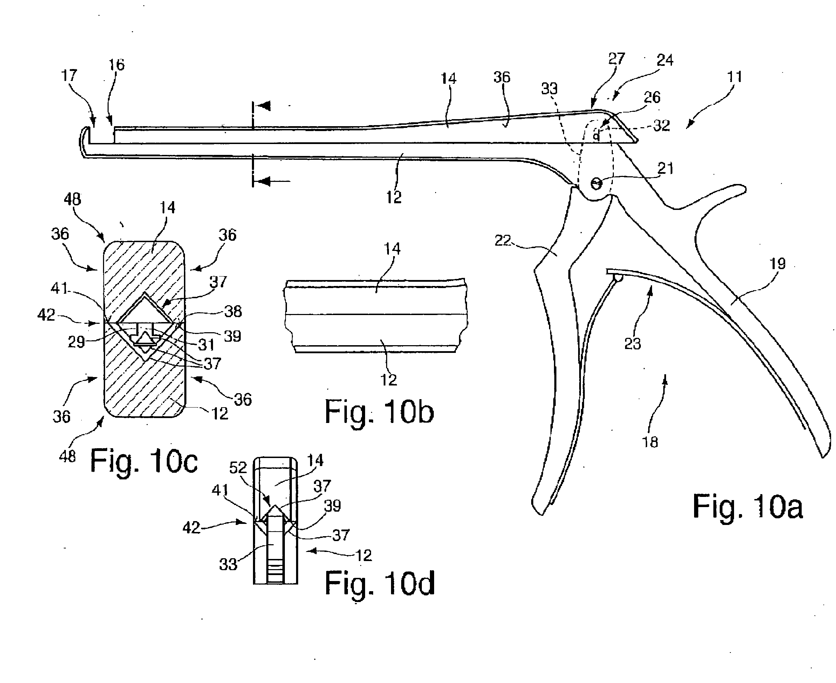

In Figur 10a ist eine alternative Ausführungsform eines chirurgischen Instrumentes 11 zu Figur 1 dargestellt. Bei dieser alternativen Ausführungsform ist die Gleitfläche 39 und/oder Führungsfläche 41 in einer anderen Raumrichtung als in den vorstehend beschriebenen Ausführungsformen durchbrochen. In diesem Fall ist die Gleitfläche 39 und/oder Führungsfläche 41 in Längsrichtung des Hauptteils 12 und des bewegbaren Teils 14 durchbrochen. Dadurch wird ermöglicht, wie dies in Figur 10b dargestellt ist, dass eine geschlossene Außenseite 36 des Hauptteils 12 und bewegbaren Teils 14 ausgebildet ist. Die dadurch gebildete Aussparung 37 in Längsrichtung des Hauptteils 12 und bewegbaren Teils 14 ist als Kanal 52 ausgebildet, dessen Querschnitt beispielsweise in Figur 10c dargestellt ist. Eine Seitenansicht auf das bewegbare Teil 14 und das Hauptteil 12 ist in Figur 10c dargestellt. Diese Aussparung 37 erstreckt sich von einem proximalen Ende bis zu einem distalen Ende des chirurgischen Instrumentes 11 beziehungsweise proximalen Ende des bewegbaren Teils 14 und Hauptteils 12 bis zum distalen Ende des bewegbaren Teils 14 und Hauptteils 12. Gemäß der Darstellung in Figur 10c ist die Aussparung 37 dachförmig ausgebildet beziehungsweise stellt eine dreiecksförmige Aussparung 37 dar. Analoges gilt für die Aussparung 37 im Hauptteil 12. Diese Aussparungen 37 im Hauptteil und bewegbaren Teil erstrecken sich bis zu den Führungen 27, so dass auch diese Bereiche beim Spülen mit einem Reinigungsmedium gesäubert werden können.

Alternativ zur dargestellten Ausführungsform kann entweder nur im bewegbaren Teil 14 oder nur im Hauptteil 12 oder alternierend mit einem zumindest geringfügigen Überlappungsbereich jeweils eine Aussparung 37 vorgesehen sein. Im jeweils äußeren Randbereich des bewegbaren Teils 14 und des Hauptteils 12 wird, wie in Figur 10c dargestellt ist, ein schmaler Spalt 42 zwischen der verbleibenden Gleitfläche 39 und Führungsfläche 41 ausgebildet. Dieser Spalt 42 ist wieder von innen heraus spülbar. Ebenso kann vorgesehen sein, dass an der Außenseite jeweils zur Trennebene zwischen der Gleitfläche 39 und der Führungsfläche 41 einseitig oder spiegelbildlich zur Trennebene wiederum eine Schrägfläche 43 angeordnet sein kann.

Damit eine Reinigung innerhalb des Führungsbereiches bei dieser Ausführungsform ebenfalls möglich ist, ist bevorzugt vorgesehen, dass zumindest die Vertiefung 29 oder der Führungszapfen 31 ebenfalls eine in Längsrichtung sich erstreckende Aussparung 37 aufweist. Bevorzugt weisen sowohl die Vertiefung 29 als auch der Führungszapfen 31 eine Aussparung 37 auf, so dass auch dieser Bereich gereinigt werden kann. Dies ist beispielhaft in Figur 10c dargestellt.

## Patentansprüche

1. Chirurgisches Instrument, insbesondere Schiebeschaftinstrument, mit einem Hauptteil (12) und einem relativ dazu bewegbaren Teil (14),
mit einem an dem Hauptteil (12) angeordneten Handgriff (18), der einen feststehenden Griff (19) und einen das bewegbare Teil (14) betätigbaren Griff (22) aufweist,
mit zumindest einer Führung (27), durch welche das bewegbare Teil (14) relativ zum Hauptteil (12) zwischen einer Ausgangsposition (24) und einer Arbeitsposition bewegbar ist, und das bewegbare Teil (14) durch eine Gelenkverbindung (26) am distalen Ende geführt ist, wobei
eine am bewegbaren Teil (14) angeordnete und zum Hauptteil (12) weisende Gleitfläche (39), die entlang einer am Hauptteil (12) angeordneten und zur Gleitfläche (39) weisenden Führungsfläche (41) verschiebbar geführt ist, **dadurch gekennzeichnet,**
**dass** die Gleitfläche (39) und die Führungsfläche (41) zur Bildung eines Spaltes (42) zumindest abschnittsweise aufeinander aufliegen und,
**dass** zumindest die Gleitfläche (39) oder die Führungsfläche (41) quer zur Längsrichtung des bewegbaren Teils und des Hauptteils auf eine Breite reduziert ist, oder
**dass** zumindest die Gleitfläche (39) oder die Führungsfläche (41) durch zumindest eine quer zur Längserstreckung des Hauptteils (12) und bewegbaren Teils (14) sich erstreckende Aussparung (37) durchbrochen ist, so dass die Spülbarkeit des Spalts (42) zwischen der Gleitfläche (39) und der Führungsfläche (41) zur Reinigung des chirurgischen Instrumentes verbessert wird.

2. Chirurgisches Instrument nach Anspruch 1, **dadurch gekennzeichnet, dass** zumindest die Gleitfläche (39) oder die Führungsfläche (41) zur Bildung eines spülbaren Spaltes (42) einander zugeordnet sind.

3. Chirurgisches Instrument nach Anspruch 1, **dadurch gekennzeichnet, dass** an einer Außenseite (36) des Hauptteils (12) oder des bewegbaren Teils (14) oder an der Außenseite (36) des Hauptteils (12) und der Außenseite des bewegbaren Teils (14) eine an die Führungsfläche (41) oder Gleitfläche (39) angrenzende Schrägfläche (43) vorgesehen ist, welche zumindest abschnittsweise zur Längsmittelachse des Hauptteils (12) und des bewegbaren Teils (14) geneigt ist.

4. Chirurgisches Instrument nach Anspruch 1, **dadurch gekennzeichnet, dass** an jeder Außenseite (36) des Hauptteils (12) und des bewegbaren Teils (14) die Gleitfläche (39) und an die Führungsfläche (41) angrenzend eine Schrägfläche (43) vorgesehen ist.

5. Chirurgisches Instrument nach Anspruch 4, **dadurch gekennzeichnet, dass** die Schrägfläche (43) spiegelsymmetrisch zur Längsmittelachse des bewegbaren Teils (14) und Hauptteils (12) oder spiegelsymmetrisch zum Spalt (42) ausgebildet ist.

6. Chirurgisches Instrument nach der Anspruch 1, **dadurch gekennzeichnet, dass** an nur einer Außenseite (36) der Gleitfläche (39) oder der Führungsfläche (41) oder an der Außenseite (36) der Gleitfläche (39) und der Führungsfläche (41) angrenzend eine Schrägfläche (43) vorgesehen ist, welche sich im Wesentlichen über die gesamte Breite des bewegbaren Teils (14) oder Hauptteils (12) erstreckt.

7. Chirurgisches Instrument nach Anspruch 1, **dadurch gekennzeichnet, dass** zumindest die Gleitfläche (39) oder die Führungsfläche (41) durch eine in Längsrichtung des Hauptteils (12) und bewegbaren Teils (14) sich erstreckende Aussparung (37) durchbrochen ist.

8. Chirurgisches Instrument nach Anspruch 1, **dadurch gekennzeichnet, dass** die Aussparung (37) wellenförmig, dreiecksförmig, rechteckförmig, trapezförmig, bogensegmentförmig oder sägezahnförmig ausgebildet ist.

9. Chirurgisches Instrument nach Anspruch 1, **dadurch gekennzeichnet, dass** die Aussparung (37) durch zwei benachbart zueinander angeordnete kegelstumpfförmige, pyramidenstumpfförmige, halbkugelförmige oder bogensegmentförmige Erhebungen (50) ausgebildet ist.

10. Chirurgisches Instrument nach einem der vorhergehenden Ansprüche, **dadurch gekennzeichnet, dass** zumindest eine Aussparung (37) oder eine Schrägfläche (43) an eine Führung (27) angrenzt.

11. Chirurgisches Instrument nach Anspruch 10, **dadurch gekennzeichnet, dass** die Aussparung (37) oder Schrägfläche (43) durch eine T-förmige Vertiefung (29) und einen Führungszapfen (31) oder durch eine Gelenkverbindung (26) ausgebildet ist.

## Claims

1. Surgical instrument, in particular a sliding shaft instrument, with a main part (12) and a relative moveable part (14),
with a handle (18) arranged on the main part (12), the former having a fixed grip (19) and a grip (22) for actuating the moveable part (14),
with at least one guide (27) through which the moveable part (14) relative to the main part is moveable between an initial position (24) and a working position, and the moveable part (14) is guided at the distal end with a hinge joint (26),
with a sliding surface (39) pointing to the main part (12) and arranged on the moveable part (14), the sliding surface (39) being slidable along the guide surface (41) pointing to the sliding surface (39) and arranged on the main part (12), **characterised in,**
**that** the sliding surface (39) and the guide surface (41) are arranged at least partially with each other for the formation of a gap (42), and
**that** at least the sliding surface (39) or the guide surface (41) is reduced transversely to the longitudinal direction of the movable part and the mainpart to a width or
at least the sliding surface (39) or the guide surface (41) is opened by at least one recess (37) extending transversely across the longitudinal extent of the main part (12) and moveable part (14), improving the formation of the gap (42) between the sliding surface (39) and the guide surface (41) for the cleaning of the surgical instrument.

2. Surgical instrument according to claim 1, **characterized in that** at least the sliding surface (39) or the guide surface(41) are positioned to each other for the formation of a washable gap (42).

3. Surgical instrument according to claim 1, **characterised in that** on an external side (36) of the main part (12) or the moveable part (14), or on an external side (36) of the main part (12) and the external side of the moveable part (14), a bevel (43) is provided bordering the guide surface (41) or sliding surface (39), which is sloped in at least portions to the longitudinal centre line of the main part (12) and the moveable part (14).

4. Surgical instrument according to claim 1, **characterised in that** a bevel (43) is provided on each external side (36) of the main part (12) and the moveable part (14), the sliding surface (39) and bordering the guide surface (41),

5. Surgical instrument according to claim 4, **characterised in that** the bevel (43) is constructed mirror-symmetrically about the longitudinal centre line of the moveable part (14) and main part (12), or mirror-symmetrically about the gap (42).

6. Surgical instrument according to claim 1, **characterised in that** on only one external side (36) of the sliding surface (39) or the guide surface (41), or on an external side (36) of the sliding surface (39) and bordering the guide surface (41), a bevel (43) is provided, which extends substantially over the whole width of the moveable part (14) or main part (12).

7. Surgical instrument according to claim 1, **characterised in that** at least the sliding surface (39) or the guide surface (41) is opened by a recess (37) extending in a longitudinal direction of the main part (12) and moveable part (14).

8. Surgical instrument according to claim 1, **characterised in that** the recess (37) is formed in an undulating, triangular, rectangular, trapezoidal or sawtooth form.

9. Surgical instrument according to claim 1, **characterised in that** the recess (37) is formed by two elevations (50) arranged neighbouring each other, in truncated cone form, in frustropyramidal form, in hemispherical form, or in the form of a crescent segment.

10. Surgical instrument according to claim 1, **characterised in that** at least one recess or a bevel (43) borders a guide (27).

11. Surgical instrument according to claim 10, **characterised in that** the recess (37) or bevel (43) is formed by a T-shaped indentation (29) and a pilot pin (31), or by a hinge joint (26).

## Revendications

1. Instrument chirurgical, notamment instrument à coulisse, avec une partie principale (12) et une partie mobile (14) pouvant être déplacée par rapport à celle-ci,
avec une poignée (18) qui est disposée sur la partie principale (12) et présente une branche fixe (19) et une branche (22) pouvant actionner la partie mobile (14),
avec au moins un guide (27) grâce auquel la partie mobile (14) peut être déplacée par rapport à la partie principale (12) entre une position initiale (24) et une position de travail, et grâce auquel la partie mobile (14) est guidée au moyen d'un joint articulé (26) à l'extrémité distale,
une surface de glissement (39) disposée sur la partie mobile (14) et orientée vers la partie principale (12) étant déplaçable de manière guidée le long d'une surface de guidage (41) disposée sur la partie principale (12) et orientée vers la surface de glissement (39), **caractérisé en ce que**
la surface de glissement (39) et la surface de guidage (41) reposant au moins partiellement l'une sur l'autre en vue de former une fente (42),
au moins la surface de glissement (39) ou la surface de guidage (41) est réduite à une largeur donnée transversalement au sens longitudinal de la partie mobile et de la partie principale, ou
au moins la surface de glissement (39) ou la surface de guidage (41) est traversée par au moins un évidement (37) s'étendant de manière transversale à la dimension longitudinale de la partie principale (12) et de la partie mobile (14)-de sorte que la rinçabilité de la fente (42) entre la surface de glissement (39) et la surface de guidage (41) en vue du nettoyage de l'instrument chirurgical se trouve améliorée.

2. Instrument chirurgical selon la revendication 1, **caractérisé en ce qu'**au moins la surface de glissement (39) ou la surface de guidage (41) sont agencées l'une par rapport à l'autre en vue de former une fente rinçable (42).

3. Instrument chirurgical selon la revendication 1, **caractérisé en ce que**, sur une face extérieure (36) de la partie principale (12) ou de la partie mobile (14) ou sur la face extérieure (36) de la partie principale (12) et sur la face extérieure de la partie mobile (14), est prévu un plan incliné (43) avoisinant la surface de guidage (41) ou la surface de glissement (39), lequel est incliné au moins partiellement par rapport à l'axe central longitudinal de la partie principale (12) et de la partie mobile (14).

4. Instrument chirurgical selon la revendication 1, **caractérisé en ce que** la surface de glissement (39) est prévue sur chaque face extérieure (36) de la partie principale (12) et de la partie mobile (14) et qu'un plan incliné (43) est prévu de manière avoisinante à la surface de guidage (41).

5. Instrument chirurgical selon la revendication 4, **caractérisé en ce que** le plan incliné (43) est formé de manière symétrique à l'axe central longitudinal de la partie mobile (14) et de la partie principale (12) ou de manière symétrique à la fente (42).

6. Instrument chirurgical selon la revendication 1, **caractérisé en ce qu'**il est prévu de manière adjacente à une seule face extérieure (36), à savoir à celle de la surface de glissement (39) ou de la surface de guidage (41), ou de manière adjacente à la face extérieure (36) de la surface de glissement (39) et de la surface de guidage (41) un plan incliné (43) qui s'étend essentiellement sur toute la largeur de la partie mobile (14) ou de la partie principale (12).

7. Instrument chirurgical selon la revendication 1, **caractérisé en ce qu'**au moins la surface de glissement (39) ou la surface de guidage (41) est traversée par un évidement (37) s'étendant dans le sens longitudinal de la partie principale (12) et de la partie mobile (14).

8. Instrument chirurgical selon la revendication 1, **caractérisé en ce que** l'évidement (37) est réalisé de forme ondulée, triangulaire, rectangulaire, trapézoïdale, en segment d'arc, ou en dents de scie.

9. Instrument chirurgical selon la revendication 1, **caractérisé en ce que** l'évidement (37) est formé
par deux saillies (50) voisines en forme de cône tronqué, de pyramide tronquée, d'hémisphère ou de segment d'arc.

10. Instrument chirurgical selon l'une des revendications précédentes, **caractérisé en ce qu'**au moins un évidement (37) ou un plan incliné (43) avoisine un guide (27).

11. Instrument chirurgical selon la revendication 10, **caractérisé en ce que** l'évidement (37) ou le plan incliné (43) est formé par un creux (29) en T et un tenon de guidage (31) ou par un joint articulé (26).
